# EUROPÄISCHE PATENTSCHRIFT

(11) **EP 1 337 182 B1**
(45) Veröffentlichungstag und Bekanntmachung des Hinweises auf die Patenterteilung: **26.03.2008**
(21) Anmeldenummer: 01989497.1
(22) Anmeldetag: 21.11.2001
(51) Int. Cl.: A61B 5/15, A61B 5/00

(54) **BLUTANALYSEGERÄT**
BLOOD TESTING APPARATUS
APPAREIL D'ANALYSE DU SANG

(30) Priorität: 21.11.2000 DE 10057832
(43) Veröffentlichungstag der Anmeldung: 27.08.2003
(73) Patentinhaber: Pelikan Technologies, Inc., Palo Alto CA 94303 (US)
(72) Erfinder: WURSTER, Thomas, 89522 Heidenheim (DE); MALOWANIEC, Krzysztof, 89522 Heidenheim (DE); DIEKMANN, Christoph, 89522 Heidenheim (DE)
(74) Vertreter: Harris, Ian Richard
(86) Internationale Anmeldenummer: PCT/EP2001/013514
(87) Internationale Veröffentlichungsnummer: WO 2002/041779

(56) Entgegenhaltungen:
- EP-A- 0 777 123
- EP-A- 0 964 060
- EP-A- 0 985 376
- US-A- 5 035 704
- US-A- 5 971 941
- US-A- 6 036 924

## Beschreibung

Die Erfindung betrifft ein Blutanalysegerät zur Bestimmung eines Analyten wie Fructosamin, Lactat, Cholesterol, insbesondere Glucose, an unmittelbar zuvor entnommenen Minimalstmengen von Blut eines Benutzers.

Die Erfindung befasst sich mit solchen Blutanalysegeräten, die ausgebildet sind mit einem ein Meßfeld definierenden membranartigen Testmittel, welches mit der entnommenen Minimalmenge von Blut benetzt wird und Testreagenzien umfasst, mit einer eine Auswerteelektronik umfassenden, beispielsweise optisch, vorzugsweise reflektometrisch, oder elektrochemisch arbeitenden Auswerteeinrichtung und mit einer Anzeigeeinrichtung, wobei die vorstehend erwähnten Komponenten ein als ein einziges Gerät handhabbares Komplettsystem-bilden.

Ein derartiges Analysegerät ist bspw. aus der US-Patentschrift US-A-4,787,398 bekannt. Dieses Blutzuckermeßgerät umfasst ein Grundgerät mit einer Stößelanordnung zum Auslenken eines Stechelements und mit einer Auswerteeinrichtung und einer Anzeigeeinrichtung. Für jede Messung muß an dem Grundgerät eine auswechselbare Einheit angeordnet werden, welche das Stechelement und ein mit Blut zu benetzendes Testmittel in Form eines Teststreifens umfasst. Diese auswechselbare Einheit wird nach jedem Gebrauch verworfen.

Hiervon ausgehend liegt der vorliegenden Erfindung die Aufgabe zugrunde, ein Blutanalysegerät so weiterzubilden, dass es weniger einzeln zu handhabende Komponenten aufweist und damit einfacher bedienbar und benutzerfreundlicher ist.

Ein aus EP 0 449 525 A1 bekanntes Blutanalysegerät umfasst zwar ebenfalls eine integrierte Auslösevorrichtung für ein Stechelement. Vor jeder Inbetriebnahme muß aber ein neues Stechelement manuell in die Auslösevorrichtung als Teil der Blutentnahmevorrichtung eingesetzt werden, und danach muß ein Teststreifen in das Gerät eingesetzt werden.

Auch US-A-4,627,445 zeigt ein Komplettsystem für ein Blutzuckermeßgerät im vorstehenden Sinne. Auch hier muß aber vor jeder Messung eine neue auswechselbare Einheit aus Stechelement und Testmittel aufwendig an einem Grundgerät montiert und danach demontiert werden.

Entsprechendes zeigt US-A-5,951,492. Gemäß dieser Druckschrift umfasst eine wegwerfbare Einheit eine kapillare Röhre, an deren körperabgewandtem Ende ein Teststreifen vorgesehen ist, der mit der entnommenen Minimalmenge von Blut beaufschlagt wird. Die kapillare Röhre ist an ihrem Vorderende mit einem Stechelement ausgebildet. Wiederum muß vor und nach jedem Meßvorgang eine neue wegwerfbare Einheit der soeben beschriebenen Art montiert bzw. demontiert werden. Nach einem weiteren Ausführungsbeispiel ist im Bereich der benutzerzugewandten Stirnseite des Geräts ein querverlaufender Schlitz vorgesehen, durch den eine poröse Testmembran mit Träger eingesteckt werden kann, die dann von dem Stechelement beim Stechvorgang durchstoßen wird.

US-A-5,971,941 zeigt nach einer Ausführungsform ein Komplettsystem im vorstehend geschilderten Sinne, wobei eine Kassette mit ungebrauchten streifenförmigen Testmitteln in ein Grundgerät eingesetzt und dann mittels eines Schiebers ein jeweiliges Testmittel in eine jeweilige Arbeitsposition gebracht werden kann. Über eine Auslöseeinrichtung, welche einen Teil der Blutentnahmevorrichtung bildet, wird mittels eines Stößels eine in einem jeweiligen Teststreifen untergebrachte Nadel zur Blutentnahme nach außen gestoßen, um die Hautoberfläche eines Benutzers zu durchstechen, damit kapillares Blut zur Analyse gewonnen werden kann. Nähere Ausführungen, wie die Analyse ausgeführt wird, lassen sich dieser Druckschrift nicht entnehmen. Nach einer weiteren in dieser Druckschrift beschriebenen Ausführungsform wird ein wegwerfbarer zylinderförmiger Aufsatz oder Einsatz beschrieben, welcher ein Stechelement und eine tablettenförmige Testmembran mit einer Druchtrittsöffnung für das Stechelement aufweist. Dieser Aufsatz oder Einsatz wird dann in eine Halteausnehmung einer Stößelanordung eingesetzt, welche das Stechelement zur Blutentnahme nach außen drückt. Auch hier muß wiederum vor und nach jedem Testvorgang die wegwerfbare Einheit montiert bzw. demontiert werden.

US-A-5035704 offenbart ein Blutanalysegerät gemäß dem Oberbegriff des Anspruchs 1.

Die eingangs erläuterte Aufgabe, eine bedienerfreundliche Weiterbildung eines Blutanalysegeräts der genannten Art zu schaffen, die auch eine sichere Blutversorgung des Testmittels mit möglichst geringsten Blutmengen gewährleistet, wird erfindungsgemäß dadurch gelöst, dass eine Mehrzahl von Testmitteln in das Gerät einsetzbar ist, die zur Durchführung mehrerer Messungen nacheinander in eine Arbeitsposition bringbar sind, in der sie mit der Auswerteeinrichtung zusammenwirken können, dass die Blutentnahmevorrichtung ebenfalls eine Mehrzahl von Stechelementen aufweist, und dass bei Positionierung eines jeweiligen Testmittels in der Arbeitsposition ein Stechelement durch das Testmittel hindurchstoßbar und in die Hautoberfläche eines Benutzers einstechbar ist, die an einer der Arbeitsposition zugeordneten Stechposition am Gerät angelegt ist, so daß aus der Hautoberfläche austretendes Blut direkt das Testmittel beaufschlagen kann, dass die Testmittel auf einem gegenüber einem Gehäusekörper des geräts beweglichen Träger angeordnet und mit diesem in den Gehäusekörper einsetzbar sind, und dass der Träger ringartig ausgebildet und um das Ringzentrum drehbar ist, und die Testmittel in die Arbeitsposition zu bringen.

Es soll also erfindungsgemäß eine Montage oder Demontage vor und nach jedem Test-, Meß- oder Analysevorgang vermieden werden. Aus diesem Grund ist in dem Blutanalysegerät eine Mehrzahl von Testmitteln und vorzugsweise eine dieser Anzahl genau entsprechende Anzahl von Stechelementen vorgesehen, die nacheinander in die erwähnte Arbeitsposition bringbar sind und dann durch Betätigen oder Auslösen der Blutentnahmevorrichtung mit dieser zusammenwirken. Ein in der Arbeitsposition befindliches Stechelement wird dann durch das membranartige Testmittel hindurch in die Hautoberfläche eines Benutzers eingestochen, so dass die dabei gewonnene Minimalstmenge unmittelbar das membranartige Testmittel benetzt, ohne zuvor kapillare Röhren oder Spalte durchdringen zu müssen, die ihrerseits wiederum Blutvolumen beanspruchen. Die Testmittel können dann nacheinander durch Drehen des Trägers in die Arbeitsposition bzw. aus der Arbeitsposition heraus in eine Speicher- und Entsorgungsposition gebracht werden. Um die Testmittel bzw. Stechmittel in die Arbeitsposition zu bringen und die Stechmittel zu betätigen, sind an sich beliebige mechanische oder elektromotorische Schalt- und Antriebsmittel denkbar. Die Anzahl der vorzugsweise als Einheit handhabbaren Testmittel und vorteilhafterweise auch der Stechmittel beträgt vorzugsweise 5 bis 75, insbesondere 14-28. Die Anzahlen 14 und 28 entsprechen einem 2 bzw. 4-Wochenrhythmus wenn pro Tag eine Analyse genommen wird.

Nach der Auswertung und Anzeige des Ergebnisses der Analyse, bspw. des Blutzuckergehalts, wird das jeweilige Testmittel aus der Arbeitsposition gebracht und dabei vorzugsweise gleich das nächstfolgende Testmittel in die Arbeitsposition gebracht.

Vor diesem Vorgang könnte das Stechelement wieder aus dem Testmittel herausgezogen werden. Es erweist sich indessen als vorteilhaft, wenn das Stechelement nach dem Stechvorgang in dem Testmittel verbleibt und zusammen mit diesem aus der Arbeitsposition entfernbar ist, um ein neues Testmittel dort anzuordnen. Das Stechelement kann auch soweit zurückgezogen werden, dass es nicht über eine Fingerauflagefläche des Gerätes vorsteht. Dies ist aber nicht zwingend erforderlich.

Nach einer weiteren Ausführungsform der Erfindung ist es denkbar, dass das Stechelement schon vor dem Stechvorgang mit dem membranartigen Testmittel verbunden ist und zusammen mit diesem in das Gerät einsetzbar und in die Arbeitsposition verlagerbar ist. Das Stechelement kann dabei bereits in das membranartige Testmittel eingesteckt oder durch dieses hindurch gesteckt sein.

Nach einem Stech- und Meßvorgang können gebrauchte Stechelemente und Testmittel einzeln oder gemeinsam auswerfbar sein oder sie können in eine Speicher- und Errtsorgungsposition gebracht werden.

Dabei sind die Testmittel vorteilhafterweise so auf dem Träger angeordnet, dass ihre jeweilige Flächennormale in radialer Richtung in Bezug auf den drehbaren Träger verläuft.

Vorzugsweise ist ein Schutz gegen Verschmutzung, Kontamination und Einwirkung von Feuchtigkeit vorzusehen. Der Träger kann hierfür vorteilhafterweise kassettenförmig geschlossen ausgebildet sein. Der Träger kann dann über fenster- oder blendenartig verschließbare und freigebbare Öffnungen verfügen, um mit dem Stoßmechanismus zusammenzuwirken und das Stechelement zur Ausführung des Stechvorgangs nach außen treten zu lassen bzw. Blut zu den Testmitteln gelangen zu lassen. Als weiteren Schutz, insbesondere vor Luftfeuchtigkeit, könnten die Testmittel alternativ oder zusätzlich durch folienartige Abdeckmittel überfangen sein, die dann in der Arbeitsposition entfernbar sind.

Innerhalb der Ringform ist dann vorteilhafterweise die Blutentnahmevorrichtung mit den mehreren Stechelementen untergebracht. Hierzu ist es denkbar, dass innerhalb der Ringform eine Auslösevorrichtung, wei sie an sich bekannt und bspw. in den eingangs erwähnten Druckschriften beschrieben ist, untergebracht ist. Beispielsweise ist eine stößelartige Stoßanordnung verwirklicht, welche auf die körperabgewandte Seite eines in der Arbeitsposition angeordneten Stechelements so einwirkt, dass es in die Hautoberfläche eines Benutzers eindringt. Es wäre auch denkbar, dass ein jeweiliges Stechelement in der Arbeitsposition in eine klemmende Anordnung etwa zwischen aufeinander zu bewegbaren Backen des Stoßorgans genommen wird, so dass mit Vor- und Zurückbewegen des Stoßorgans das Stechelement nach außerhalb des Geräts bewegt und wieder zurückgezogen werden kann. Jedenfalls bildet die Antriebseinheit der Blutentnahmevorrichtung, welche also ein jeweiliges Stechelement durch das membranartige Testmittel hindurch in die Hautoberfläche eines Benutzers stößt, einen Teil des Grund- oder Basisgeräts, wie auch die Auswerteeinrichtung und die Anzeigeeinrichtung. Demgegenüber bilden die membranartigen Testmittel und die Stechelemente wegwerfbare Elemente, die in einer vorbestimmten Konfiguration, etwa durch Anordnung auf einem Träger in den Gehäusegrundkörper eingesetzt werden.

Es erweist sich desweiteren als vorteilhaft, wenn die Stechelemente - wie bereits erwähnt - ebenfalls auf einem drehbaren Träger, vorzugsweise auf demselben Träger wie die Testmittel, in das Blutanalysegerät eingesetzt werden. Durch Drehen des oder der Träger wird dann ein jeweiliges Stechelement ebenfalls in Arbeitsposition gebracht, also in eine Position, wo es von dem Stoßorgan der Blutentnahmevorrichtung beaufschlagt oder von diesem in klemmender Anordnung gegriffen und zur Ausführung des Stechvorgangs schlagartig bewegt werden kann.

Es erweist sich als insgesamt vorteilhaft, wenn das Blutanalysegerät eine im wesentlichen kreisscheibenförmige Außenkontur aufweist, da es solchermaßen bequem mit den Händen des Benutzers ergriffen und gehalten werden kann.

In weiterer Ausbildung dieses Erfindungsgedankens weist das Gerät einander gegenüberliegend eine Stechposition zum Anlegen der zu stechenden Hautoberfläche und eine Auslöseposition zum Auslösen des Stechvorgangs durch manuelle Betätigung einer Auslösetaste auf.

Vorteilhafterweise wird das Gerät dann dadurch gehalten, dass ein Benutzer das Gerät mit zwei Fingern an der Stechposition und der Auslösetaste hält. Die Auslösetaste ist dabei vorteilhafterweise ergonomisch zum Ergreifen mittels des Daumens eines Benutzers ausgeformt. Sie verfügt vorzugsweise über einen Druckpunkt, der zum Auslösen des Stechvorgangs überwunden werden muß. Es erweist sich aus Sicherheitsgründen als vorteilhaft, wenn der Stechvorgang nur dann ausgelöst werden kann, wenn beide Finger ihre bestimmungsgemäße Position eingenommen haben. Dies könnte durch Berührungssensoren oder auch über einen Druckpunktmechanismus verwirklicht werden.

Es wird aber darauf hingewiesen, dass anstelle eines nadel- oder lanzettenförmigen Stechelements, welches in an sich bekannter Weise zur Ausführung des Stechvorgangs in Richtung auf die Hautoberfläche eines Benutzers vorzugsweise schlagartig bewegt wird, indem bspw. eine federvorgespannte Stoßeinrichtung ausgelöst wird, auch ein Laserstrahl verwendet werden kann. Die hierzu erforderliche Laserlichtquelle gehört dann zu den nicht wegwerfbaren Systemkomponenten des Blutanalysegeräts. Auch bei dieser Lösung kann ein jeweiliges Testmittel mit einer Öffnung versehen sein, durch welche der Laserstrahl hindurchtreten kann.

Nach einem weiteren Erfindungsgedanken kann das Blutanalysegerät nach Art einer Armbanduhr ausgebildet sein, d.h. es kann einen einem Armbanduhrengehäuse nachempfundenen Gehäusegrundkörper aufweisen. Eine Sichtseite des Blutanalysegeräts kann dann ein Ziffernblatt wie bei einer an sich bekannten Uhr aufweisen oder eine digitale Anzeigeeinrichtung. Die digitale Anzeigeeinrichtung kann für die Anzeige der Uhrzeit und/oder weiterer Funktionen und gegebenfalls auch für die Anzeige von Daten oder Informationen, die durch das Blutanalysegerät ermittelt werden, ausgebildet sein.

Es kann sich ferner als vorteilhaft erweisen, wenn das Blutanalysegerät einen abnehmbaren, vorzugsweise aufschwenkbaren Deckel aufweist, der einen Zugang zum Inneren des Blutanalysegeräts, insbesondere zum Einsetzen bzw. Austauschen des Trägers für die Testmittel und/oder Stechelemente, aufweist. Bei der Ausbildung der äußeren Erscheinung des Blutanalysegeräts nach Art einer Armbanduhr oder auch nach Art einer Taschenuhr, kann es sich als vorteilhaft erweisen, wenn der abnehmbare oder aufschwenkbare Deckel zugleich das Ziffernblatt oder eine sonstige Zeitanzeigeeinrichtung umfasst, die dann mit dem Deckel aufgeklappt oder aufgeschwenkt wird. Nach einem weiteren Erfindungsgedanken kann dann der aufgeklappte Deckel die Einsichtnahme auf eine Anzeigeeinrichtung des Blutanalysegeräts freigeben, die sich entweder auf der nach innen gewandten Seite des aufgeklappten Deckels befinden kann oder durch das Abnehmen oder Aufschwenken des Deckels freigegeben wird. Es kann sich ferner als vorteilhaft erweisen, wenn unterhalb des ersten abnehmbaren oder aufschwenkbaren Deckels ein zweiter abnehmbarer oder aufschwenkbarer Deckel vorgesehen ist, der den Zugang zum Inneren des Blutanalysegeräts freigibt oder verschließt. Dieser zweite Deckel könnte dann auf seiner Außenseite die Anzeigeeinrichtung des Blutanalysegeräts umfassen, die zugleich einer Zeitanzeige dienen kann. Zum Auslesen der durch das Blutanalysegerät ermittelten Daten und Informationen wird der erste Deckel geöffnet, so dass ein Benutzer die Anzeigevorrichtung auf der dann freigegebenen Sichtseite des zweiten Deckels oder der Innenseite des ersten Deckels einsehen kann. Lediglich zum Auswechseln der Testmittel oder Stechelemente wird der zweite Deckel geöffnet.

Bei einer Ausbildung des Blutanalysegeräts nach Art eines Armbanduhrengehäuses erweist es sich als vorteilhaft, wenn ein Fingerauflagebereich für die Ausführung des Stechvorgangs zum Entnehmen einer Minimalstmenge von Blut an der "6-Uhr"- oder "12-Uhr"-Position oder im jeweiligen Anfügungsbereich des Uhrenarmbands vorgesehen ist. Hierdurch wird eine komfortable Bedienbarkeit erreicht, die sich auch im Hinblick auf eine gute Benetzungsfunktion positiv auswirkt, da das jeweilige Testmittel (bei Anordnung der Testmittel im wesentlichen senkrecht zur radialen Richtung) bei der Blutnahme horizontal ausgerichtet ist, was eine gleichmäßige Benetzung fördert.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung.
In der Zeichnung zeigt:
- Figur 1: eine schematische Anordnung einer ersten Ausführungsform eines erfindungsgemäßen Blutanalysegeräts;
- Figur 2: eine Schnittansicht des Blutanalysegeräts nach Figur 1;
- Figur 3: eine explosionsartige Darstellung einer zweiten Ausführungsform eines erfindungsgemäßen Blutanalysegeräts;
- Figur 4: eine explosionsartige Darstellung des Trägers für Testmittel und Stechelemente des Geräts nach Figur 3;
- Figur 5: eine perspektivische Ansicht des zusammengebauten Blutanalysegeräts nach Figur 3;
- Figur 6: eine perspektivische Ansicht einer dritten Ausführungsform eines erfindungsgemäßen Blutanalysegeräts;
- Figur 7: eine perspektivische Ansicht des Blutanalysegeräts nach Figur 6 mit aufgeklapptem ersten Deckel;
- Figur 8: eine perspektivische Ansicht des Blutanalysegeräts nach Figur 7 mit aufgeklapptem ersten und zweiten Deckel und
- Figur 9: eine perspektivische Ansicht entsprechend Figur 8 einer vierten Ausführungsform des erfindungsgemäßen Blutanalysegeräts.

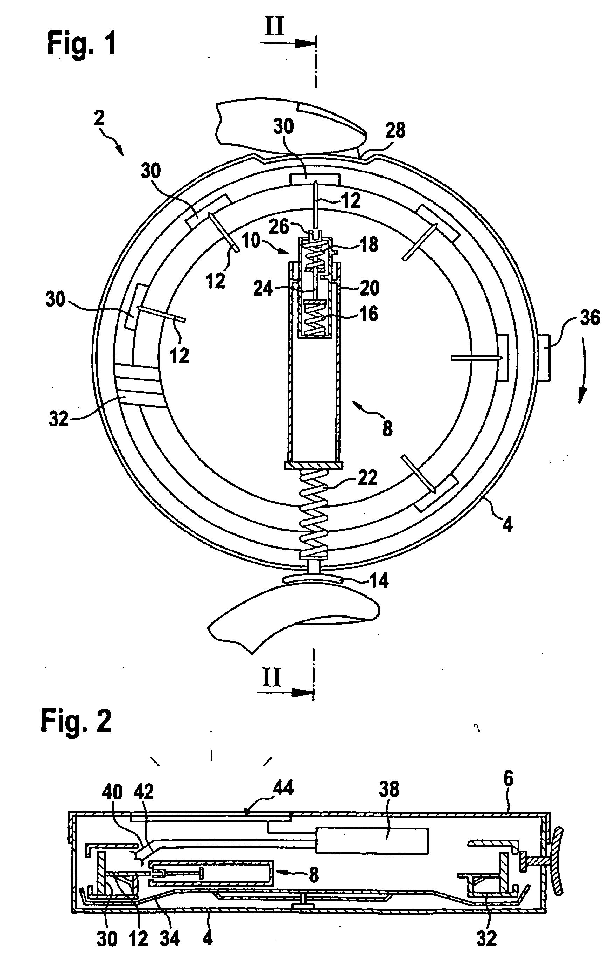

Die Figuren 1 und 2 zeigen eine schematische Ansicht eines erfindungsgemäßen Blutanalysesystems, wobei Figur 1 eine Ansicht in das Innere bei abgenommenem Deckel und Figur 2 eine schematische Schnittansicht darstellt. Das insgesamt mit dem Bezugszeichen 2 bezeichnete Blutanalysegerät in Form eines Blutzuckermeßgeräts umfasst einen Gehäusegrundkörper 4 und einen abnehmbaren Deckel 6. Im Inneren des Gehäusegrundkörpers 4 ist eine Blutentnahmevorrichtung 8 mit einem Stoßmechanismus 10 und einem Stechelement 12 in Form einer Nadel untergebracht. Die Blutentnahmevorrichtung 8 wirkt mit einer Auslösetaste 14 an der schmalen Außenseite des scheibenförmigen Gehäusegrundkörpers 4 zusammen. Der Stoßmechanismus 10 umfasst eine Stoß- und eine Rückzugsfeder 16, 18, die beide nur schematisch angedeutet sind. Über mechanische Kopplungs- und Steuermittel 20 kann durch Drücken der Auslösetaste 14 und Überwinden eines Druckpunktmechanismus 22 der Stoßmechanismus 10 ausgelöst werden, so dass unter Vorspannung der Stoßfeder 16 ein Stößel 24 nach radial außen schnellt, dabei das Stechelement 12 zwischen Backen 26 klemmend aufnimmt und nach radial nach außen stößt und unmittelbar danach unter der Wirkung der Rückzugsfeder 18 wieder geringfügig zurückzieht. Dabei dringt das Stechelement 12 kurzzeitig über die der Auslösetaste radial gegenüberliegende Fingerauflagefläche 28 an der Außenseite des Gehäusegrundkörpers 4, welche eine Stechposition definiert, vor und sticht dabei mit vorbestimmter Geschwindigkeit und Eindringtiefe in die Hautoberfläche eines Benutzers kurzzeitig ein, um dort eine Minimalstmenge von Blut austreten zu lassen.

Beim Nachaußenschnellen des Stechelements 12 wird ein membranartiges Testmittel 30, welches auf noch näher zu beschreibende Weise in der unmittelbaren Nähe hinter der Fingerauflagefläche 28 angeordnet ist, von dem Stechelement 12 durchdrungen. Das aus der Hautoberfläche austretende Blut benetzt dann unmittelbar die nach außen gewandte Oberfläche des membranartigen mit Reagenzmitteln versehenen Testmittels 30.

Wie aus den Figuren ersichtlich, ist eine Mehrzahl von Testmitteln 30 und diesen jeweils zugeordneten Stechelementen 12 vorgesehen. Die Testmittel 30 und Stechelemente 12 sind auf einem ringförmigen Träger 32 angeordnet, bspw. sind über den Umfang oder einen Teilumfang des ringsförmigen Trägers 32 acht oder zehn Paare von Testmitteln 30 und Stechelementen 12 angeordnet. Der Träger 32 ist bei abgenommenem Deckel 6 in eine komplementär ausgebildete und um das Ringzentrum drehbare Aufnahmeeinrichtung 34 einsetzbar. Es wären aber auch Ausführungsformen denkbar, bei denen der Deckel zum Einsetzen des Trägers nicht abgenommen zu werden braucht, sondern eine nach oben offene Ausnehmung zum Einsetzen eines kassettenförmig geschlossenen Trägers 32 aufweist. Hierdurch wird ein Schutz gegen Verschmutzung, Kontamination und Einwirkung von Feuchtigkeit erreicht. Der Träger 32 kann dann über fenster- oder blendenartig verschließbare und freigebbare Öffnungen verfügen, um mit dem Stoßmechanismus zusammenzuwirken und das Stechelement zur Ausführung des Stechvorgangs nach außen treten zu lassen bzw. Blut zu den Testmitteln gelangen zu lassen. Als weiteren Schutz insbesondere vor Luftfeuchtigkeit könnten die Testmittel alternativ oder zusätzlich durch folienartige Abdeckmittel überfangen sein, die dann in der Arbeitsposition entfernbar sind.

Wie aus den Figuren zu ersehen, sind die membranartigen Testmittel so angeordnet, dass sie mit ihrer Flächennormalen in radialer Richtung bzgl. des Ringzentrums angeordnet sind. Durch Betätigen eines schieberartigen Tasters 36 an der Außenseite des Gehäusegrundkörpers 4 wird die Aufnahmeeinrichtung 34 und mit ihr der darin formschlüssig gehaltene und positionierte Träger 32 in eine diskrete weitere Winkelposition gedreht, so dass aufeinanderfolgend die Paare von Testmitteln 30 und Stechelementen 12 in eine Arbeitsposition gebracht werden, in der das Stechelement mit dem Stoßmechanismus 10 zusammenwirken kann. Auf diese Weise wird das Blutzuckermeßgerät durch Einsetzen des vorzugsweise kassettenförmig ausgebildeten Trägers 32 mit einer Anzahl von bspw. zehn Testmitteln und Stechelementen für zehn Messungen konditioniert. Nach einer Messung braucht lediglich der Taster 36 betätigt zu werden, um das nächste Paar von Testmittel und Stechelement in die Arbeitsposition zu bringen. Weitere Montage- oder Demontageschritte vor und nach einem jeweiligen Meßvorgang sind nicht erforderlich. Gebrauchte Testmittel und Testelemente werden mit dem Träger in eine Speicher- oder Entsorgungsposition gebracht, welche der Arbeitsposition in Uhrzeigersinnrichtung folgt. Es wäre aber auch denkbar, einen Auswerfmechanismus vorzusehen, der ein jeweiliges gebrauchtes Paar zur Entsorgung auswirft, was jedoch als weniger bevorzugt angesehen wird, da dann unmittelbar eine sachgerechte Entsorgung stattfinden muß. Die geschützte Anordnung der gebrauchten Paare innerhalb des kassettenförmigen Trägers 32 wird demgegenüber bevorzugt. Nach Durchführung der vorbestimmten Anzahl von Analysen wird der kassettenförmige Träger entfernt und entsorgt und durch einen neuen ersetzt.

Dadurch, dass das Stechelement 12 beim Stechvorgang das membranartige Testmittel 30, vorzugsweise in dessen Zentrum durchdringt ist sichergestellt, dass das Testmittel in unmittelbarer Nähe zu der Einstichstelle an der Hautoberfläche des Benutzers positioniert ist. Das dort austretende Blut wird sofort und vor allem gleichmäßig an das Testgebiet des Testmittels abgegeben und zwar auch dann, wenn nur geringe Blutmengen zur Verfügung stehen.

Im dargestellten Fall sind die Stechelemente 12 auf dem Träger 32 so angeordnet, dass sie, wenn der Stoßmechanismus 10 auf sie einwirkt das Zentrum des Testmittels 30 durchstoßen. Hierfür kann es sich als vorteilhaft erweisen, wenn die Stechelemente 12 bereits so auf dem Träger 32 angeordnet sind, dass ihre Nadelspitze, zumindest teilweise in Dickenrichtung in das zugeordnete Testmittel 30 eingedrungen ist. Hierdurch wird eine Positionierungshilfe erreicht. Es kann in dem Testmittel 30 auch eine durchgehende Führungsöffnung vorgesehen sein. Der Durchmesser der Führungsöffnung sollte aber vorzugsweise geringer sein als der Außendurchmesser des Stechelements um zu verhindern, dass durch einen Spalt zwischen der Außenoberfläche des Stechelements und der Führungsöffnung Blut in Richtung auf die Rückseite des Testmittels hindurchdringt.

Im Inneren des Blutzuckermeßgeräts ist auch eine an sich bekannte Auswerteeinrichtung 38 vorgesehen. Eine optische, vorzugsweise reflektometrische Auswerteeinheit ist schematisch in Figur 2 angedeutet. Die Auswerteeinrichtung kann eine Lichtquelle 40 und einen Sensor 42 zur reflektometrischen Messung der Färbung der Rückseite des membranartigen Testmittels 30 umfassen, wo die Analysereaktion von in der Blutprobe enthaltener Glukose mit dem Test- oder Nachweisreagenten stattfindet (enzymatische Redox-Reaktion). Die Prinzipien einer optischen Auswerteeinrichtung sind zum Beispiel in EP-A-0 654 659 und EP-A-0 475 692 beschrieben.

Im Falle der Anwendung des elektrochemischen Meßprinzips entfällt die optische Auswerteeinrichtung. Die enzymatische Redox-Reaktion wird statt dessen über Detektion von elektrischem Strom bzw. Spannung an einer Elektrode quantifiziert (z.B. beschrieben in EP-A-0 552 223).

Die Auswerteeinrichtung 38 umfasst in an sich bekannter Weise eine Auswerteelektronik, die mit einer Anzeigeeinrichtung 44 zusammenwirkt, die beispielsweise in Form einer LCD-Anzeige das Testergebnis, etwa den Blutzuckergehalt angibt. Es könnten mittels der Auswerteeinrichtung auch weitere Auswerte-, Anzeige- und Vergleichsfunktionen mit zuvor gespeicherten Meß- oder Auswertedaten durchgeführt, gegebenenfalls gespeichert und deren Ergebnis angezeigt werden.

Das erfindungsgemäße Blutanalysegerät stellt daher ein Komplettsystem dar, welches bei der Blutzuckermessung nicht die separate Handhabung von Teststreifen oder Stechlanzetten erfordert. Das Gerät wird durch Einsetzen des kassettenförmigen Trägers 32 mit Testmitteln 30 und Stechelementen 12 für eine bestimmte Anzahl von Messungen vorbereitet, für welche keinerlei weitere Montage- oder Demontageschritte oder die separate Handhabung von weiteren Hilfsmitteln erforderlich ist.

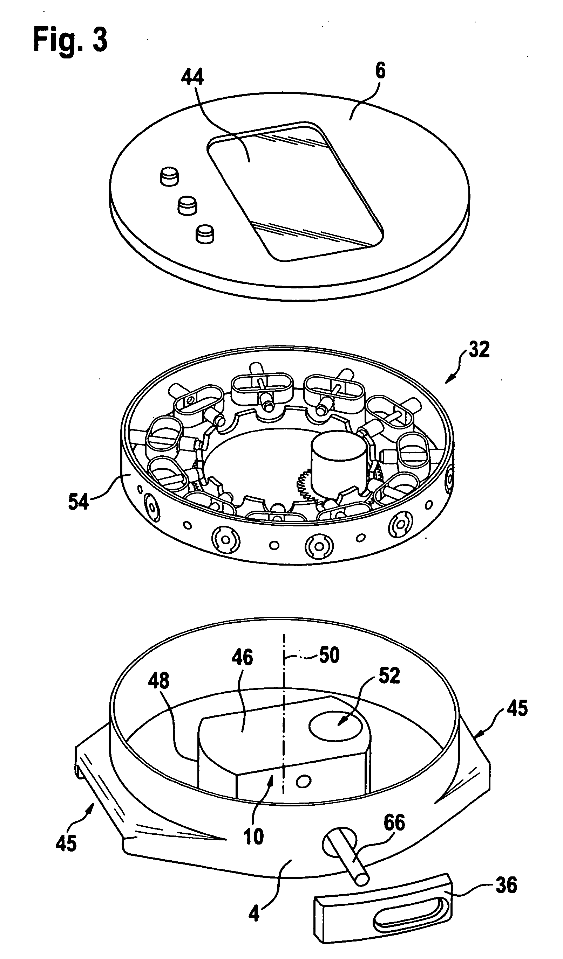

Die Figuren 3 bis 5 zeigen in verschiedenen Ansichten eine zweite Ausführungsform des erfindungsgemäßen Blutanalysegeräts, wobei der ersten Ausführungsform entsprechende Komponenten mit denselben Bezugszeichen bezeichnet sind. Danach umfasst das Blutanalysegerät einen der Grundform eines Armbanduhrengehäuses nachempfundenen oder angenäherten Gehäusegrundkörper 4, wobei die Dimensionen, insbesondere die Höhe des Gehäusegrundkörpers 4, gegenüber üblichen Armbanduhrgehäusen vergrößert sein können. Des weiteren angedeutet sind Montagebereiche 45 für einen insbesondere federnden Steg eines üblichen Armbandes. Im Inneren des Gehäusegrundkörpers 4 ist ein domförmiges Zentriermittel 46 dargestellt, welches in der Draufsicht zwar quaderförmig erscheint, jedoch zwei teilkreisförmige Seitenabschnitte 48 aufweist, die konzentrisch zu einer Drehachse 50 ausgebildet sind und beim Einsetzen eines Trägers 32 für Testmittel 30 und Stechelemente 12 eine Positionierhilfe bieten. Ferner ist in dem domförmigen Zentriermittel 46 ein Stellmotor 52 (im Einzelnen nicht dargestellt) untergebracht. Der Stellmotor 52 kann zum Bewegen des Trägers 32 dienen, d.h. um ein gebrauchtes Testmittel aus einer Arbeitsposition in eine Entsorgungsposition zu verbringen und gleichzeitig ein noch ungebrauchtes Testmittel in der Arbeitsposition zu positionieren. Es ist indessen nicht ausgeschlossen, dass der Stellmotor 52 auch zum Antrieb des nur schematisch dargestellten Stoßmechanismus 10 dienen kann. Die Antriebskopplung des Stellmotors 52 mit dem Träger 32 könnte beispielsweise durch eine Ritzel-, Kronrad-, Kegel- oder Winkelgetriebeverbindung zwischen einem rotierend angetriebenen Rad des Stellmotors 52 und entsprechend ausgebildeten insbesondere zahnkranzförmigen korrespondierenden Getriebemitteln an dem Träger 32 ausgebildet sein.

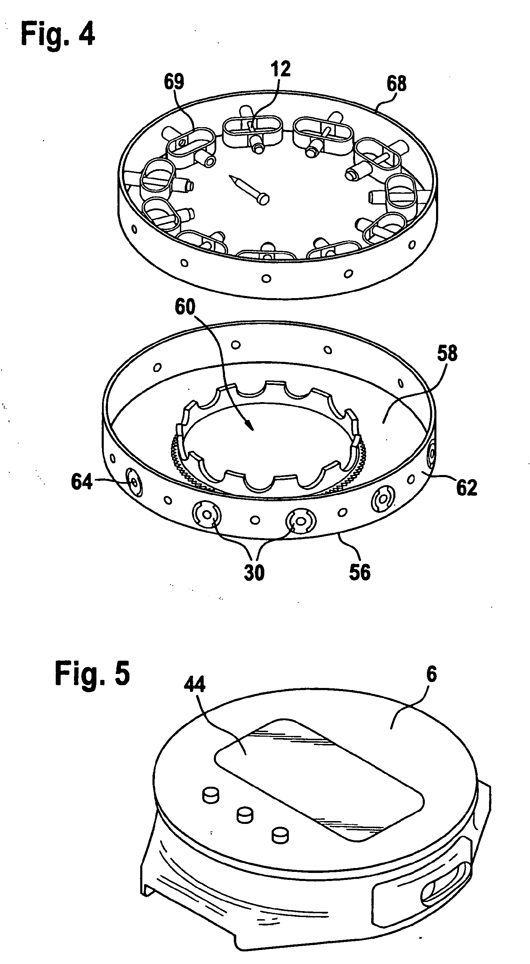

Wie in den Figuren 3 und 4 dargestellt, ist der Träger 32 in Form einer ringscheibenförmigen Kassette 54 ausgebildet. Die Kassette 54 umfasst ein Gehäuseunterteil 56 mit einem ringscheibenförmigen Bodenteil 58 mit einer kreisförmigen Durchgriffsöffnung 60 und mit am äußeren Umfang zylinderförmig verlaufendem Umfangswandabschnitt 62. In dem Umfangswandabschnitt 62 sind die Testmittel 30 in konzentrischer Anordnung um die Drehachse 50 in entsprechenden Ausnehmungen 64 vorgesehen. In dieses Gehäuseunterteil 56 ist ein formähnlich ausgebildetes Gehäuseoberteil 68 einsetzbar, welches eine der Anzahl der Testmittel 30 entsprechende Anzahl von Stechelementen 12 radial ausgerichtet umfasst. Man erkennt auch Federmittel 69, insbesondere in Form von geschlossenen Bandschlaufen, welche die Stechelemente 12 halten. Beim Einstechen in die Hautoberfläche eines Benutzers werden diese Federelemente 69 gespannt und vermögen nach dem Einstechen durch den Stoßmechanismus 10 das jeweilige Stechelement 12 wieder zurückzuziehen. Diese Anordnung der Stechelemente 12 befindet sich radial außerhalb der vorstehend erwähnten Öffnung 60 und damit radial außerhalb des domförmigen Zentriermittels 46, das zugleich den Stoßmechanismus 10, der somit radial innerhalb der Anordnung von Stechelementen 12 angeordnet ist, umfasst. Das Gehäuseunterteil 56 und das darin eingesetzte Gehäuseoberteil 68 sind drehfest miteinander gekoppelt und können als Träger 32 gemeinsam um die Achse 50 verdreht werden, um Testmittel 30 und Stechelemente 12 in die Arbeitsposition zu bringen bzw. aus der Arbeitsposition in eine Entsorgungsposition zu verbringen.

Der in Figur 3 schematisch angedeutete Taster 36 ist in Steuerverbindung mit dem Stoßmechanismus 10. Die dort als radial verlaufend angedeutete Steuerstange 66 verläuft entweder oberhalb oder unterhalb des Trägers 32. Wie erwähnt könnte aber die Ansteuerung des Stoßmechanismus 10 auch motorisch und dabei vorzugsweise elektrisch gesteuert erfolgen.

Schließlich umfasst das Blutanalysegerät einen Deckel 6, der dem Ziffernblatt einer elektronischen Uhr nachempfunden sein kann und eine Anzeigeeinrichtung 44, beispielsweise in Form einer LCD-Anzeige, aufweisen kann. Dieser Deckel bildet dann die Sichtseite des Blutanalysegeräts, wie aus Figur 5 ersichtlich.

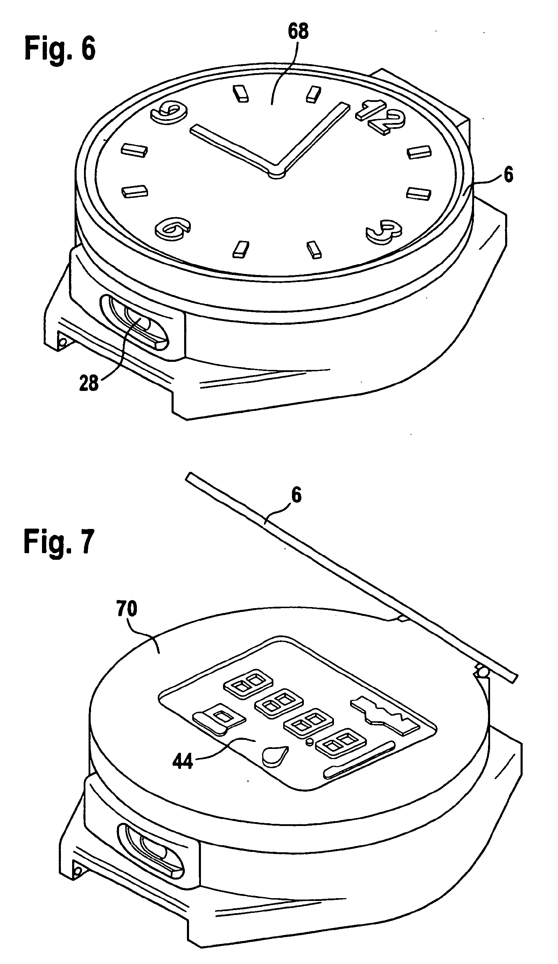

Figur 6 zeigt eine Figur 5 entsprechende perspektivische Ansicht eines Blutanalysegeräts mit einem Uhrenzifferblatt 68 an der Sichtseite eines schwenkbar angelenkten Deckels 6. Zu erwähnen sei noch, dass eine Fingerauflagefläche 28 an der "6-Uhr-Position" in Bezug auf das Ziffernblatt 68 vorgesehen ist, welche die Arbeitsposition bildet, in der die Hautoberfläche von dem Stechelement beim Auslösen des Stoßmechanismus 10 kurzzeitig durchdrungen wird. Diese Anordnung erweist sich insofern als vorteilhaft, als bei der Ausführung des Stechvorgangs der Benutzer die Hand (stehend) auf den Bauch legen kann und dann den Daumen der anderen Hand an der Fingerauflagefläche 28 positioniert. Wenn in dieser Position der Stechvorgang ausgelöst wird, so ist das membranartige Testmittel 30 in der Arbeitsposition im wesentlichen horizontal angeordnet und die Minimalstmenge an Blut vermag das Testmittel der Schwerkraft folgend zu benetzen.

Figur 7 zeigt das Blutanalysegerät nach Figur 6 mit nach oben geschwenktem ersten Deckel 6, so dass die Sicht auf die Oberseite eines zweiten Deckels 70 freigegeben ist, wo nach dieser Ausführungsform die Anzeigeeinrichtung 44 für das Blutanalysegerät vorgesehen ist. Die Anzeigeeinrichtung 44 für das Blutanalysegerät ist daher räumlich von dem Ziffernblatt 68 bzw. der Anzeigevorrichtung für die Zeit getrennt. Selbstverständlich könnte die Anzeigeeinrichtung 44 zugleich der Anzeige der Zeit dienen.

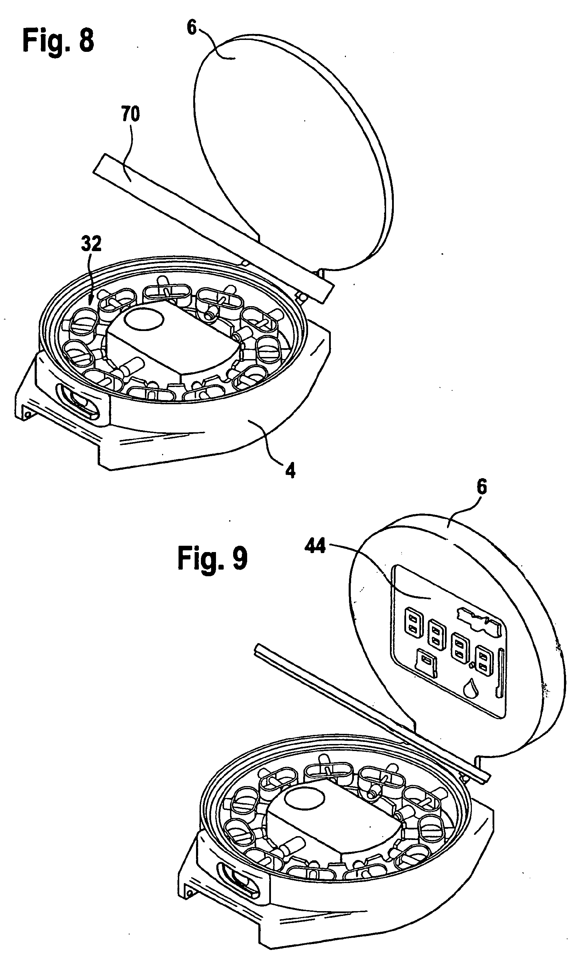

Figur 8 zeigt das Blutanalysegerät nach Figur 7 mit ebenfalls hochgeklapptem zweitem Deckel, so dass ein Zugang in den Gehäusegrundkörper 4 zum Einsetzen und Entnehmen einer Trägerkassette möglich ist.

Schließlich zeigt Figur 9 eine Figur 8 entsprechende perspektivische Ansicht einer weiteren Ausführungsvariante, wonach die Anzeigeeinrichtung 44 für die Blutanalyse auf der Innenseite des ersten Deckels 6 vorgesehen ist.

## Patentansprüche

1. Blutanalysegerät mit einer ein Stechelement (12) aufweisenden Blutentnahmevorrichtung (8), mit einem ein Meßfeld definierenden membranartigen Testmittel (30), mit einer eine Auswerteelektronik umfassenden Auswerteeinrichtung (38) und mit einer Anzeigeeinrichtung (44), die ein als ein einziges Gerät handhabbares Komplettsystem bilden, wobei
eine Mehrzahl von Testmitteln (30) in das Gerät einsetzbar ist, die zur Durchführung mehrerer Messungen nacheinander in eine Arbeitsposition bringbar sind,
die Blutentnahmevorrichtung (8) ebenfalls eine Mehrzahl von Stechelementen (12) aufweist,
bei Positionierung eines jeweiligen Testmittels (30) in der Arbeitsposition ein Stechelement (12) durch das Testmittel (30) hindurchstoßbar und in die Hautoberfläche eines Benutzers einstechbar ist, die an einer der Arbeitsposition zugeordneten Stechposition (28) am Gerät angelegt ist, so dass aus der Hautoberfläche austretendes Blut direkt das Testmittel (30) beaufschlagen kann,
die Testmittel (30) auf einem gegenüber einem Gehäusekörper (4) des Geräts beweglichen Träger (32) angeordnet und mit diesem in den Gehäusekörper (4) einsetzbar sind, **dadurch gekennzeichnet, dass** die Testmittel in der Arbeitsposition mit der Auswerteeinrichtung zusammenwirken können, und
der Träger (32) ringartig ausgebildet und um das Ringzentrum drehbar ist, um die Testmittel in die Arbeitsposition zu bringen.

2. Blutanalysegerät nach Anspruch 1, wobei das Stechelement (12) nach dem Stechvorgang in dem Testmittel (30) verbleibt und zusammen mit diesem aus der Arbeitsposition entfernbar ist, um ein neues Testmittel dort anzuordnen.

3. Blutanalysegerät nach Anspruch 1 oder 2, wobei das Stechelement (12) schon vor dem Stechvorgang mit dem Testmittel (30) handhabbar verbunden ist und zusammen mit diesem in das Gerät einsetzbar und in die Arbeitsposition verlagerbar ist.

4. Blutanalysegerät nach Anspruch 1, 2 oder 3, wobei gebrauchte Stechelemente (12) und Testmittel (30) auswerfbar oder in eine Speicher- und Entsorgungsposition bringbar sind.

5. Blutanalysegerät nach Anspruch 4, wobei die Testmittel (30) so auf dem Träger (32) angeordnet sind, dass die jeweilige Flächennormale der Testmittel in radialer Richtung verläuft.

6. Blutanalysegerät nach einem der vorstehenden Ansprüche, wobei innerhalb der Ringform des trägers die Stechvorrichtung untergebracht ist.

7. Blutanalysegerät nach einem der vorstehenden Ansprüche, wobei die Stechmittel (12) auf einem gegenüber einem Gehäusekörper (4) drehbaren Träger (32) angeordnet und mit diesem in den Gehäusekörper (4) des Geräts einsetzbar sind.

8. Blutanalysegerät nach Anspruch 7, wobei die Stechelemente (12) und die Testmittel (30) auf demselben Träger (32) angeordnet sind.

9. Blutanalysegerät nach einem der vorstehenden Ansprüche, wobei das Gerät eine im wesentlichen kreisscheibenförmige Außenkontur aufweist.

10. Blutanalysegerät nach einem der vorstehenden Ansprüche, wobei das Gerät einander gegenüberliegend die Stechposition (28) zum Anlegen der zu stechenden Hautoberfläche und eine Auslöseposition zum Auslösen des Stechvorgangs durch manuelle Betätigung einer Auslösetaste (14) aufweist.

11. Blutanalysegerät nach Anspruch 10, wobei das Gerät **dadurch** gehalten werden kann, dass ein Benutzer mit zwei Fingern das Gerät an der Stechposition (28) und der Auslösetaste (14) hält.

12. Blutanalysegerät nach einem der vorstehenden Ansprüche, mit einer Sicherheitseinrichtung, die ein Auslösen des Stechvorgangs erst bei bestimmungsgemäßer Handhabung des Geräts erlaubt.

13. Blutanalysegerät nach Anspruch 10, 11 oder 12, wobei die Auslösetaste (14) ergonomisch zum Ergreifen mittels des Daumens eines Benutzers ausgeformt ist.

14. Blutanalysegerät nach Anspruch 10, 11, 12 oder 13, wobei die die Auslösetaste (14) über einen Druckpunkt verfügt, der zum Auslösen der Stechvorrichtung überwunden werden muß.

15. Blutanalysegerät nach einem der vorstehenden Ansprüche, wobei die Anzahl der als Einheit handhabbaren Testmittel 5 bis 75, vorzugsweise 14-28, beträgt.

16. Blutanalysegerät nach einem der vorstehenden Ansprüche, wobei der Träger (32) ein erstes Gehäuseteil (56) für die Testmittel (30) und ein zweites Gehäuseteil (68) für die Stechelemente (12) umfasst, die ineinander einsetzbar sind.

17. Blutanalysegerät nach einem der vorstehenden Ansprüche, wobei der Träger eine mittige Ausnehmung (60) aufweist, welche den Stoßmechanismum (10) und gegebenenfalls ein elektromotorisches Antriebsmittel (52) für den Träger (32) und/oder den Stoßmechanismum (10) aufweist.

18. Blutanalysegerät nach einem der vorstehenden Ansprüche, wobei der Träger (32) ein Federmittel (69) zum Zurückziehen eines Stechelements (12) aus der Hautoberfläche des Benutzers aufweist.

## Claims

1. A blood analysis apparatus comprising a blood extraction device (8) having a piercing element (12), a membrane-like test means (30) defining a measurement field, an evaluation device (38) comprising an electronic evaluation means and a display device (44), which form a complete system which can be handled as a single apparatus, wherein
a plurality of test means (30) can be fitted into the apparatus, which are moveable successively into an operating position for carrying out a plurality of measurement operations,
the blood extraction device (8) also has a plurality of piercing elements (12),
when a respective test means (30) is positioned in the operating position a piercing element (12) can be pushed through the test means (30) and can pierce the surface of the skin of a user, which is placed against the apparatus at a piercing position associated with the operating position so that blood issuing from the surface of the skin can impinge directly on the test means (30), and
the test means (30) are disposed on a carrier (32) which is moveable with respect to a housing body (4) of the apparatus and can be inserted with said carrier into the housing body (4).
**characterised in that** the test means can co-operate with the evaluation device in the operating position and
the carrier (32) is of a ring configuration and is rotatable about the centre of the ring to bring the test means into the operating position.

2. A blood analysis apparatus according to claim 1 wherein the piercing element (12) remains in the test means (30) after the piercing operation and can be removed together with said test means from the operating position in order to position a new test means there.

3. A blood analysis apparatus according to claim 1 or claim 2 wherein the piercing element (12) is already handleably connected to the test means (30) prior to the piercing operation and can be inserted together therewith into the apparatus and can be moved into the operating position.

4. A blood analysis apparatus according to claim 1, claim 2 or claim 3 wherein spent piercing elements (12) and test means (30) can be ejected or can be moved into a storage and disposal position.

5. A blood analysis apparatus according to claim 4 wherein the test means. (30) are disposed on the carrier (32) in such a way that the respective surface normal of the test means extends in the radial direction.

6. A blood analysis apparatus according to one of the preceding claims wherein the piercing device is disposed within the ring shape of the carrier.

7. A blood analysis apparatus according to one of the preceding claims wherein the piercing means (12) are arranged on a carrier (32) rotatable with respect to a housing body (4) and can be inserted with said carrier into the housing body (4) of the apparatus.

8. A blood analysis apparatus according to claim 7 wherein the piercing elements (12) and the test means (30) are arranged on the same carrier (32).

9. A blood analysis apparatus according to one of the preceding claims wherein the apparatus has an external contour substantially in the form of a circular disc.

10. A blood analysis apparatus according to one of the preceding claims wherein the apparatus has in mutually opposite relationship a piercing position (28) for application of the skin surface to be pierced and a triggering position for triggering the piercing operation by manual actuation of a triggering button (14).

11. A blood analysis apparatus according to claim 10 wherein the apparatus can be held by a user holding the apparatus at the piercing position (28) and the triggering button (14) with two fingers.

12. A blood analysis apparatus according to one of the preceding claims comprising a safety device which permits triggering of the piercing operation only when the apparatus is handled correctly.

13. A blood analysis apparatus according to claim 10, claim 11 or claim 12 wherein the triggering button (14) is ergonomically shaped to be gripped by the thumb of a user.

14. A blood analysis apparatus according to claim 10, claim 11, claim 12 or claim 13 wherein the triggering button (14) has a pressure point which has to be overcome for triggering the piercing device.

15. A blood analysis apparatus according to one of the preceding claims wherein the number of test means which can be handled as a unit is 5 to 75, preferably 14-28.

16. A blood analysis apparatus according to one of the preceding claims wherein the carrier (32) comprises a first housing portion (56) for the test means (30) and a second housing portion (68) for the piercing elements (12), which housing portions can inserted one into the other.

17. A blood analysis apparatus according to one of the preceding claims wherein the carrier has a central opening (60) which has the thrust mechanism (10) and optionally an electric-motor drive means (52) for the carrier (32) and/or the thrust mechanism (10).

18. A blood analysis apparatus according to one of the preceding claims wherein the carrier (32) has a spring means (69) for retracting a piercing element (12) from the surface of the skin of the user.

## Revendications

1. Appareil d'analyse de sang comprenant un dispositif de prélèvement de sang (8) présentant un élément de piquage (12), un moyen de test (30) en forme de membrane et définissant un champ de mesure, un dispositif d'analyse (38) comprenant une électronique d'analyse et un dispositif d'affichage (44), qui forme un système complet manipulable sous forme d'un appareil unique,
une pluralité de moyens de test (30) pouvant être insérés dans l'appareil, lesquels peuvent être amenés de façon successive dans une position de travail pour la réalisation de plusieurs mesures,
le dispositif de prélèvement de sang (8) présentant également une pluralité d'éléments de piquage (12),
un élément de piquage (12) pouvant être traversé par le moyen de test (30) en cas de positionnement d'un moyen de test (30) respectif dans la position de travail et pouvant être piqué dans la surface de peau d'un utilisateur, laquelle est placée sur l'appareil en une position de piquage (28) associée à la position de travail, de sorte que du sang sortant directement de la surface de la peau peut alimenter directement le moyen de test (30),
les moyens de test (30) étant disposés sur un support (32) mobile par rapport à un corps de boîtier (4) de l'appareil et pouvant être insérés avec ce support dans le corps de boîtier (4), **caractérisé en ce que**
les moyens de test peuvent coopérer dans la position de travail avec le dispositif d'analyse, et
le support (32) est conçu en forme de bague et peut tourner autour du centre de la bague afin d'amener les moyens de test dans la position de travail.

2. Appareil d'analyse de sang selon la revendication 1, l'élément de piquage (12) restant dans le moyen de test (30) après le piquage et pouvant être enlevé conjointement avec celui-ci de la position de travail, afin d'y disposer un nouveau moyen de test.

3. Appareil d'analyse de sang selon la revendication 1 ou 2, l'élément de piquage (12) étant relié de façon manipulable déjà avant l'opération de piquage avec le moyen de test (30), pouvant être inséré conjointement avec ce moyen dans l'appareil et déplacé dans la position de travail.

4. Appareil d'analyse de sang selon la revendication 1, 2 ou 3, des éléments de piquage (12) et des moyens de test (30) utilisés pouvant être jetés ou amenés dans une position de stockage et d'évacuation.

5. Appareil d'analyse de sang selon la revendication 4, les moyens de test (30) étant disposés sur le support (32) de telle sorte que la perpendiculaire respective à la surface des moyens de test est disposée dans la direction radiale.

6. Appareil d'analyse de sang selon l'une des revendications précédentes, le dispositif de piquage étant logé à l'intérieur de la forme annulaire du support.

7. Appareil d'analyse de sang selon l'une des revendications précédentes, le moyen de piquage (12) étant disposé sur un support (32) pouvant tourner par rapport à un corps de boîtier (4) et pouvant être inséré avec ce support dans le corps de boîtier (4) de l'appareil.

8. Appareil d'analyse de sang selon la revendication 7, les éléments de piquage (12) et les moyens de test (30) étant disposés sur le même support (32).

9. Appareil d'analyse de sang selon l'une des revendications précédentes, l'appareil présentant un contour extérieur sensiblement en forme de disque circulaire.

10. Appareil d'analyse de sang selon l'une des revendications précédentes, l'appareil présentant opposées l'une à l'autre la position de piquage (28) pour le placement de la surface de peau à piquer et une position de déclenchement pour le déclenchement de l'opération de piquage par l'actionnement manuel d'une touche de déclenchement (14).

11. Appareil d'analyse de sang selon la revendication 10, l'appareil pouvant être maintenu par le fait qu'un utilisateur maintient avec deux doigts l'appareil sur la position de piquage (28) et sur la touche de déclenchement (14).

12. Appareil d'analyse de sang selon l'une des revendications précédentes, comprenant un dispositif de sécurité, qui permet un déclenchement de l'opération de piquage seulement si l'appareil est manipulé correctement.

13. Appareil d'analyse de sang selon la revendication 10, 11 ou 12, la touche de déclenchement (14) étant formée de façon ergonomique pour être saisie au moyen du pouce d'un utilisateur.

14. Appareil d'analyse de sang selon la revendication 10, 11, 12 ou 13, la touche de déclenchement (14) disposant d'un point de pression qui doit être surmonté pour le déclenchement du dispositif de piquage.

15. Appareil d'analyse de sang selon l'une des revendications précédentes, le nombre de moyens de test pouvant être manipulés sous forme d'unité étant 5 à 75, de préférence de 14 à 28.

16. Appareil d'analyse de sang selon l'une des revendications précédentes, le support (32) comprenant une première partie de boîtier (56) pour les moyens de test (30) et une seconde partie de boîtier (68) pour les éléments de piquage (12), qui peuvent être insérés les uns dans les autres.

17. Appareil d'analyse de sang selon l'une des revendications précédentes, le support présentant une cavité (60) centrale, laquelle présente un mécanisme de percussion (10) et éventuellement un moyen d'entraînement (52) par moteur électrique pour le support (32) et/ou le mécanisme de percussion (10).

18. Appareil d'analyse de sang selon l'une des revendications précédentes, le support (32) présentant un moyen à ressort (69) pour le retrait d'un élément de piquage (12) de la surface de peau de l'utilisateur.
